# EUROPEAN PATENT APPLICATION

(11) **EP 1 457 565 A1**
(43) Date of publication of application: **15.09.2004**
(21) Application number: 02790839.1
(22) Date of filing: 24.12.2002
(51) Int. Cl.: C12N 15/869, C12N 15/70, C12N 1/21, C12N 5/10, C12N 7/01, C12N 7/02

(54) **DOUBLE-STRANDED CYCLIC DNA CAPABLE OF PROLIFERATING AS ARTIFICIAL E. COLI CHROMOSOME**

(30) Priority: 25.12.2001 JP 2001391300
(71) Applicant: Evec Incorporated, Sapporo-shi, Hokkaido 060-0042 (JP)
(72) Inventor: TAKADA, Kenzo, Sapporo-shi, Hokkaido 001-0904 (JP); KANDA, Teru, Sapporo-shi, Hokkaido 001-0024 (JP)
(74) Representative: Bublak, Wolfgang, Dr.
(86) International application number: PCT/JP2002/013426
(87) International publication number: WO 2003/056023

(57) **Abstract**

(PROBLEM) To establish a system enabling modification and proliferation of EB virus circular DNA in *Escherichia coli* and large-scale production of recombinant EB virus virions in the cells.

(Means for Resolution) The present invention enables modification and proliferation of a circular EB virus genome derived from Akata cells in *Escherichia coli* by inserting a DNA sequence of a bacterial artificial chromosome (BAC) into a circular EB virus DNA in Akata cells via homologous recombination. The recombinant EB virus can be produced in a large quantity by introducing the resulting genomic DNA into Akata cells.

## Description

### Technical Field

The present invention relates to a technique enabling the proliferation of Epstein-Barr virus (which will hereinafter be called "EB virus" or "EBV") circular DNA, particularly circular EB virus DNA derived from Akata cells and its recombinants, by using *Escherichia coli;* and large-scale production of infectious EB viruses using the technique.

### Background Art

Numbers of EB virus-producing cells, such as Akata cells, B95-8 cells, and P3HR-1 cells, are known. Among these cell lines, Akata cells are suitable for large-scale production of EB viruses, because infectious EB virus production can be induced only by adding anti-human immunoglobulin antibody to culture medium (Takada K, Int. J. Cancer, 33, 27-32(1984); Takada K. and Ono Y, J. Virol, 63, 445-449(1989)).

A clone missing an EB virus genome, which has been obtained by long-term subculture, (which will hereinafter be called "EB virus-negative Akata cells") reacquires a virus-producing ability by infecting it with an EB virus again. The virus production can be induced by anti-human immunoglobulin antibody treatment in the resultant cell clone (Japanese Patent Laid-Open No. Hei 7-115966). A recombinant EB virus, having a marker gene inserted by homologous recombination in Akata cells having a wild type EB virus, can be used, as a mixture with wild-type virus, to infect EB virus-negative Akata cells. The infected cells can then be subjected to drug selection, resulting in the establishment of Akata cells having only a recombinant EB virus. These cells produce a large amount of the pure recombinant virus after anti-human immunoglobulin antibody treatment. At present, however, the EB virus DNA derived from Akata cells can be proliferated only within the Akata cells, and the modification of the genome can only be carried out within the Akata cells. This method is time-consuming and is therefore disadvantageous.

On the other hand, circular EB virus DNA derived from B95-8 cell line, which is another EB virus-producing cell line, has been cloned into a bacterial artificial chromosome of *Escherichia coli,* and it can be proliferated in *Escherichia coli* (U.S. Patent No. 6,291,246). This system is advantageous, because mutation can be introduced efficiently into the viral genome by utilizing highly efficient homologous recombination in *Escherichia coli.* At present, however, there exists no cell line suitable for large-scale production of infectious viruses of B95-8 strain, which has been modified and proliferated in *Escherichia coli.*

Only one currently-existing system is composed of introducing EB virus DNA of the B95-8 strain, which has been proliferated in *Escherichia coli,* into human 293 cells, then exogenously expressing a virus immediate-early gene BZLF1 by transfection, and thereby inducing virus production (H-J Delecluse, W. Hammer-schmidt, J. Clin. Pathol: Mol Pathol 2000; 53:270-279). This system is not suitable for large-scale production of infectious viruses, because the way of inducing virus production is cumbersome and the obtained virus titer is low. The possibility of producing viruses by introducing EB virus DNA derived from B95-8 cells into Akata cells has not yet been investigated.

### Disclosure of the Invention

An object of the present invention is to establish a system enabling modification and proliferation of circular DNA of an EB virus in *Escherichia coli* and permitting intracellular large-scale production of EB virus virions.

The present inventors inserted the DNA sequence of a bacterial artificial chromosome (which will hereinafter be called "BAC") into circular EB virus DNA derived from Akata cells via homologous recombination: and enabled the modification and proliferation of the genome of circular EB virus genome derived from Akata cells in *Escherichia coil.* The inventors also found that the introduction of the genomic DNA into Akata cells enabled large-scale production of EB viruses. Based on these observations, the present inventors completed the present invention.

In the present invention, there is thus provided a double-stranded circular DNA obtainable by inserting a vector fragment of bacterial artificial chromosome derived from Escherichia coil F factor into a circular Epstein-Barr virus genome. This circular Epstein-Barr virus genome is preferably derived from Akata cells. The double-stranded circular DNA may have at least one marker gene functioning in cells inserted therein. Moreover, in this double-stranded circular DNA, a desired gene may be incorporated into the EB virus genome, preferably by homologous recombination.

In the present invention, there is also provided *Escherichia coli* having any one of the above-described double-stranded circular DNAs as a bacterial artificial chromosome clone.

In the present invention, there is also provided a method of using any one of the above-described double-stranded circular DNAs as a vector.

In the present invention, there are also provided EBNA1-positive Akata cells constitutively expressing a viral protein EBNA1, which have been prepared by introducing a viral gene EBNA1 into EB virus-negative Akata cells.

In the present invention, there are also provided Akata cells obtainable by transfecting a double-stranded circular DNA, which has a desired gene incorporated into the EB virus genome via homologous recombination, into EB virus-positive or-negative Akata cells, or EBNA1-positive Akata cells constitutively expressing a viral protein EBNA1.

In the present invention, there is also provided a method of producing an EB virus, which comprises subjecting these Akata cells to anti-immunoglobulin treatment; or an EB virus produced by these Akata cells.

In the present invention, there is also provided a method of producing an Epstein-Barr virus, which comprises:
(a) a step of introducing any one of the above-described double-stranded circular DNAs into Epstein-Barr virus-positive Akata cells by transfection;
(b) a step of producing the Epstein-Barr virus by subjecting the Akata cells of (a) to anti-immunoglobulin treatment;
(c) a step of introducing the Epstein-Barr virus produced in the step (b) into Epstein-Barr virus-negative Akata cells;
(d) a step of selecting the Akata cells having any one of the above-described double-stranded circular DNAs from the Akata cells into which the virus has been introduced in the step (c); and
(e) a step of subjecting the Akata cells selected in the step (d) to anti-immunoglobulin treatment.

In the present invention, there is also provided a method of producing an Epstein-Barr virus, which comprises:
(a) a step of introducing any one of the above-described double-stranded circular DNAs into Epstein-Barr virus-positive Akata cells by transfection;
(b) a step of isolating cell clones which have lost a wild-type Epstein-Barr virus from the Akata cells of the step (a); and
(c) a step of subjecting the cell clones isolated in the step (b) to anti-immunoglobulin treatment.

The circular EB virus to be used in the present invention belongs to a group of herpesvirus having known double stranded DNA. Herpesviruses have been used for various investigations with similar specific aims to those of the present invention (J. Clin. Pathol; Mol Pathol 2000; 53:270-279, etc.).

There exists an infinite number of EB virus strains, including those in Akata cells, B95-8 cells or P3HR-1 cells. According to the restriction enzyme mapping of Akata EB virus genome, it is a prototype EB virus free of deletion. Genomes derived from any EB strains can be used as the source of EB virus genome, and that derived from Akata cells is especially preferred in the present invention.

Akata cell line is a known human lymphoma-derived cell line, and infectious EB virus production can be induced by adding anti-human immunoglobulin antibody to the cells (Takada K, Int. J. Cancer, 33, 27-32(1984); Takada K. and Ono Y, J. Virol, 63, 445-449(1989)).

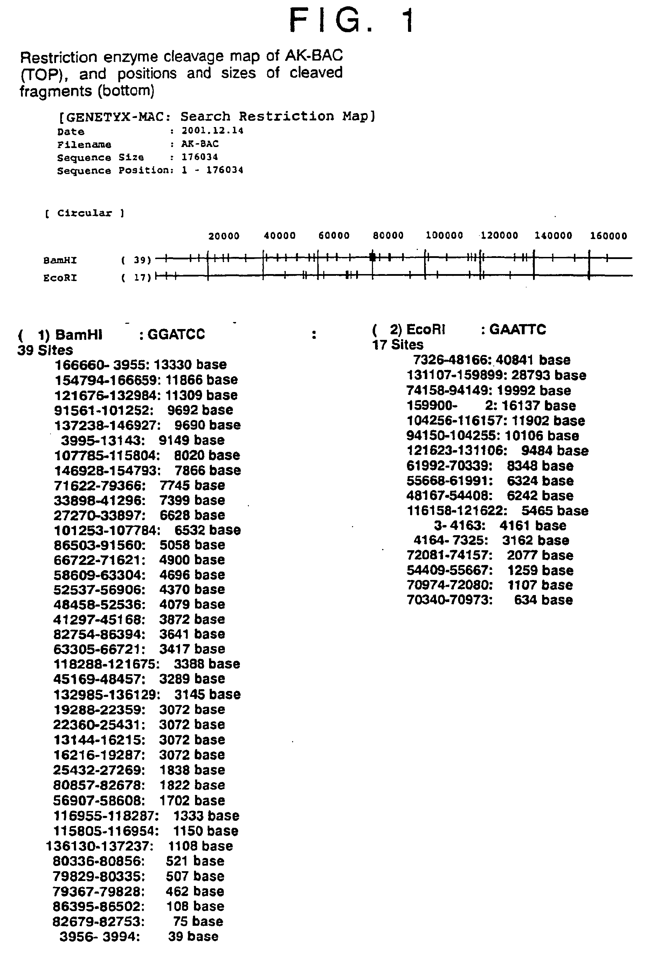

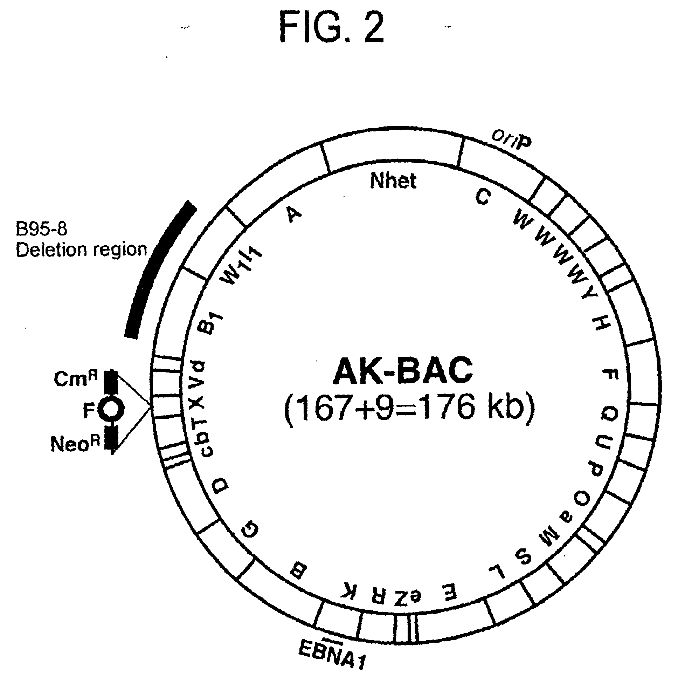

The double-stranded circular DNA of the present invention is a 176 kb double-stranded circular plasmid, designated "AK-BAC" hereinafter. The map of AK-BAC is shown in FIGS. 1 and 2. FIG. 2, which is a cleavage map of BamHI, indicates that AK-BAC has a sequence derived from an *Escherichia coli* F factor (indicated by "F" in this diagram) inserted in the BamHI X fragment. AK-BAC also has a chloramphenicol resistant gene (Cm^{R}), and it can thus proliferate as a single copy plasmid in Escherichia coil. FIG. 2 also indicates that the double-stranded circular DNA of the present invention has a neomycin-resistant gene (Neo^{R}) which works in human cells.

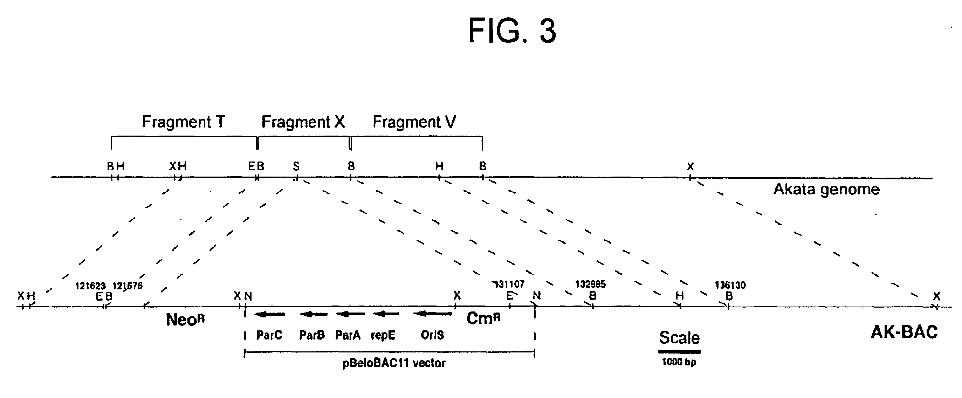

Any DNA fragment enabling replication and segregation of the resultant DNA as a single copy plasmid in Escherichia coil can be used as a vector fragment to be inserted into EB virus DNA. For example, those derived from a bacteriophage P1 or *Escherichia coli* F factor are known, and especially it is already known that pBeloBAC11 which is a vector derived from *Escherichia coli* F factor functions well in the present invention. An example of a double-stranded circular EB virus DNA (AK-BAC) having this BAC vector fragment incorporated therein is illustrated in FIG. 3 (bottom). In the AK-BAC of FIG. 3 (bottom), a neomycin resistant gene (Neo^{R}), a sequence derived from *Escherichia coli* F factor (OriS, repE, ParA, ParB, ParC), and a chloramphenicol resistant gene (Cm^{R}) have been inserted into the SmaI site (S) within the BamHI X fragment of the EBV genome.

Any known marker genes can be used, depending on various purposes. Examples include neomycin resistant gene (Neo^{R}), hygromycin resistant gene, and XGPRT gene.

The double-stranded circular DNA of the present invention can easily be modified and proliferated after being transferred into *Escherichia coli* as a BAC clone. Although there is no particular limitation imposed on the gene to be incorporated, genes with between 10 to 170 kb sizes are preferred. The bacterial artificial chromosome DNA having a desired gene inserted therein can be introduced into Akata cells. The double-stranded circular DNA of the present invention can therefore be used as a vector. If these Akata cells are subjected to anti-immunoglobulin treatment, a recombinant EB virus having the gene of interest can be produced in large-scale.

The EB virus DNA having the inserted bacterial artificial chromosome DNA in the present invention can be produced in large-scale without changing the circular form by introducing it into *Escherichia coli* and culturing the resultant *Escherichia coli* to a large quantity. Moreover, it is possible to readily produce a recombinant EB virus by modifying the DNA in *Escherichia coli* via homologous recombination, and by introducing the circular viral DNA into Akata cells by transfection.

The present invention enables convenient and large-scale production of recombinant EB viruses. By incorporating an exogenous gene into an EB virus, an exogenous gene product can be expressed in the infected cells. The present invention therefore enables using EB viruses as vectors for gene therapy. In addition, the infected cells can be used for the production of various gene products of interest.

### Brief Description of the Drawings

- FIG. 1: is a restriction enzyme (BamHI, EcoRI) cleavage map of the double- stranded circular DNA (AK-BAC) of the present invention.
- FIG. 2: is a schematic view of the structure of the double-stranded circular DNA (AK-BAC) of the present invention, in which alphabets indicate the fragments generated by the restriction enzyme BamHI;
- FIG. 3: illustrates the main restriction enzyme cleavage sites in the vicinity of BamHI X fragment of EBV genome in Akata cells (FIG. 3, top) and the main restriction enzyme cleavage sites in the vicinity of BamHI X fragment of AK-BAC after completion of homologous recombination (FIG.3 bottom), in which B, E, H, N, S and X represent the recognition sites of restriction enzymes BamHI, EcoRI, HindIII, NotI, SmaI and XbaI, respectively;
- FIG. 4: is a schematic illustration showing the processes of preparing the double-stranded circular DNA of the present invention and producing recombinant EB viruses;
- FIG. 5: illustrates the result of Southern blotting analysis of Akata cells, harboring circular EB virus DNA with the inserted BAC vector fragment, obtained by using a BamHI X fragment as a probe (Example 1);
- FIG. 6: illustrates the result of agarose gel electrophoresis of AK-BAC DNA, which has been prepared in a large-scale by proliferating *Escherichia coli* and digested with either BamHI or EcoRI (Example 1). An arrow indicates a band generated by incomplete digestion; and
- FIG. 7: illustrates the result of agarose gel electrophoresis of EcoRI-digested DNA of AK-BAC which has a GFP gene inserted by homologous recombination utilizing RecE/RecT in *Escherichia coli* (Example 2), in which the first lane shows the result prior to homologous recombination (before modification) while the other three lanes show the result after homologous recombination (after insertion of GFP).

### Best Mode for Carrying out the Invention

All the patents and references expressly cited in this specification are incorporated in this specification by citation. Details described in Japanese Patent Application 2001-391300 which becomes a base for priority claim of the present application are all incorporated in this specification by citation.

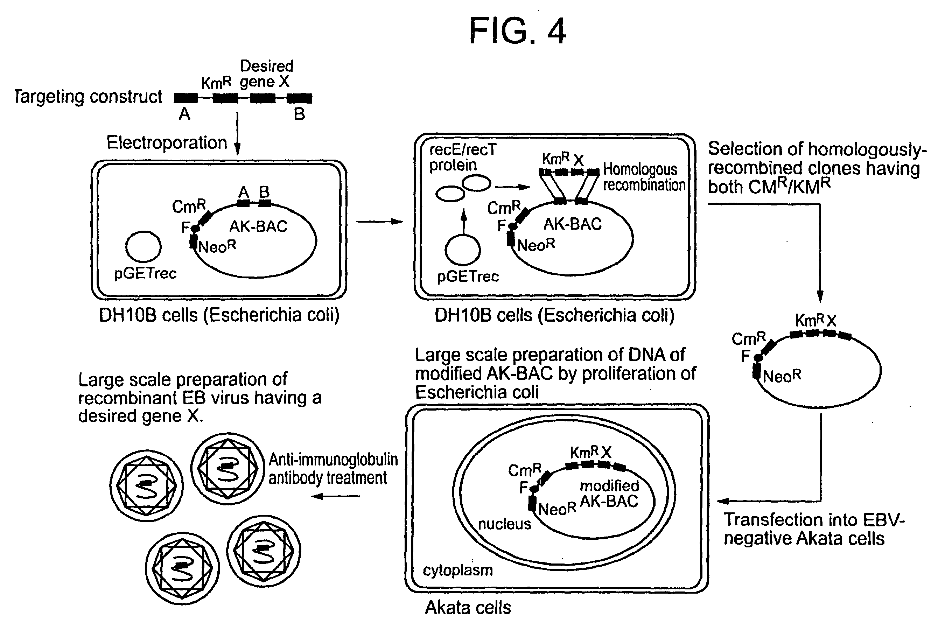

The present invention will hereinafter be described specifically by Examples. It should however be borne in mind that the present invention is not limited by them. The operation of these Examples is shown schematically in FIG. 4. In these Examples, a GFP gene is employed as a desired gene X.

### Example 1

In this Example, a BAC vector sequence was incorporated in a circular EB virus DNA in Akata cells.

A DNA fragment comprising BamHI-digested EB virus DNA fragments, T, X, and V was subcloned into an ordinarily-used cloning vector PUC119. A neomycin resistant gene (Neo^{R}) and a BAC vector fragment derived from pBeloBAC11 vector (product of Genome Systems) were inserted into the BamHI-X fragment region. As illustrated in FIG.3, the BAC vector comprises OriS, repE, ParA, ParB and ParC, which enable the maintenance of the vector as a artificial chromosome (BAC), and a chloramphenicol resistant gene (Cm^{R}).

The resultant plasmid DNA was then cut into a linear piece by using a restriction enzyme HindIII, and 20 micrograms of the linear DNA was introduced into Akata cells having wild-type circular EB virus DNA (5 × 10⁶ cells) by electroporation, followed by cultivation at 37°C for 2 days under the conditions of 5% CO₂ and saturated humidity. Two days later, the cells were suspended in medium containing 375 µg/ml of neomycin and seeded in 96-well plates so that each well would contain 10000 cells. Half of the culture medium was replaced with fresh neomycin-containing culture medium every 5 days. Three weeks later, proliferating cells that appeared in 17 wells were subjected to anti-immunoglobulin antibody treatment to induce virus production.

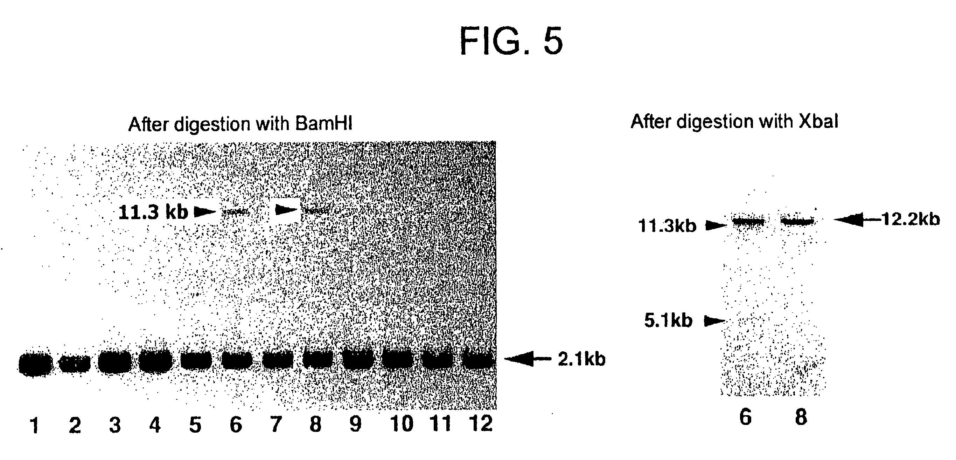

The produced viruses were subjected to Southern blotting analysis by using a BamHI X fragment as a probe, and cell clones containing the homologously-recombined EBV genome were searched. The results are shown in FIG. 5. The result of BamHI-digestion(FIG.5, left) revealed that clones 6 and 8 had, in addition to a band (about 2.1 kv) derived from a wild-type EBV, a band (about 11.3 kB) which was expected to appear when homologous recombination occurred.

Further analysis using Xbal-digested DNAs of clones 6 and 8 revealed that only clone 6 had, in addition to a band (12.2 kb) derived from a wild-type EBV, bands (11.2 kb and 5.1 kb) which were expected to appear by the correct homologous recombination(FIG.5, right). Clone 8, on the other hand, turned out to have, in addition to a band (about 12.2 kb) derived from a wild -type EBV, a band of 10.5 kb, which was probably derived from non-homologous recombination. Clone 6 was therefore confirmed to contain an EB virus genome having the inserted BAC vector DNA as well as a wild-type EB virus genome.

By using 100 ng of the whole genomic DNA extracted from the clone 6 cells, DH10B competent cells were transformed, and numbers of chloramphenicol resistant colonies (that is, colonies expressing a drug resistance marker of the BAC vector) were obtained. Six obtained colonies were proliferated, and plasmid DNAs were extracted.

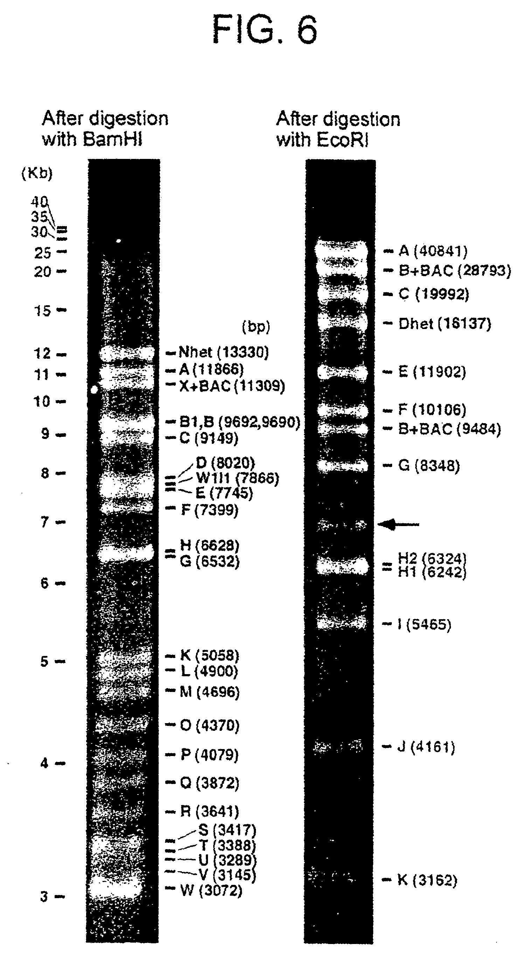

The agarose gel electrophoresis of the BamHI-or EcoRI-digested DNAs of these 6 clones revealed that all the digested DNAs exhibited the identical band patterns. The results of one clone are shown in FIG. 6. The expected cleavage pattern (FIG. 1) based on the EBV restriction enzyme map in the Akata cells is shown on the right side of each electrophoresis picture. As is apparent from this diagram, these agarose electrophoresis patterns coincide with the patterns expected from the restriction enzyme map of EB virus DNA in the Akata cells.

This BAC clone will hereinafter be called "AK-BAC". Forty micrograms of circular DNA of AK-BAC was prepared from 1 liter of bacterial culture.

### Example 2

This Example shows the possibility of rapid modification of an EB virus genome cloned into AK-BAC by using a highly-efficient homologous recombination method in *Escherichia coli.* In this Example, a gene of fluorescent protein "GFP" derived from luminous jellyfish was inserted into the region deleted in B95-8 strain (the region not necessary for virus production) of EB virus genome by homologous recombination. The method reported by Ioannou, et al. (Gene Therapy, 6, 442-447(1999)) was employed as a homologous recombination method.

A template consisting of an expression unit of the GFP gene (promoter + GFP gene + polyA signal) and a kanamycin resistant gene (Km^{R}) working in *Escherichia coli* was PCR-amplified by using synthetic primers having sequences homologous to those of the region deleted in B95-8 strain of EB virus genome on their 5' ends. The obtained PCR product was used as a targeting construct.

DH10B cells transformed by the AK-BAC obtained in Example 1 and then transformed further by pGETrec (an expression plasmid of recombinases RecE and RecT) were prepared. The cells were made competent after the induction of RecE/RecT expression.

The resulting competent cells were transformed by the targeting construct described above.

The colonies resistant to both chloramphenicol (Cm^{R}) and kanamycin (Km^{R}) were selected and then screened.

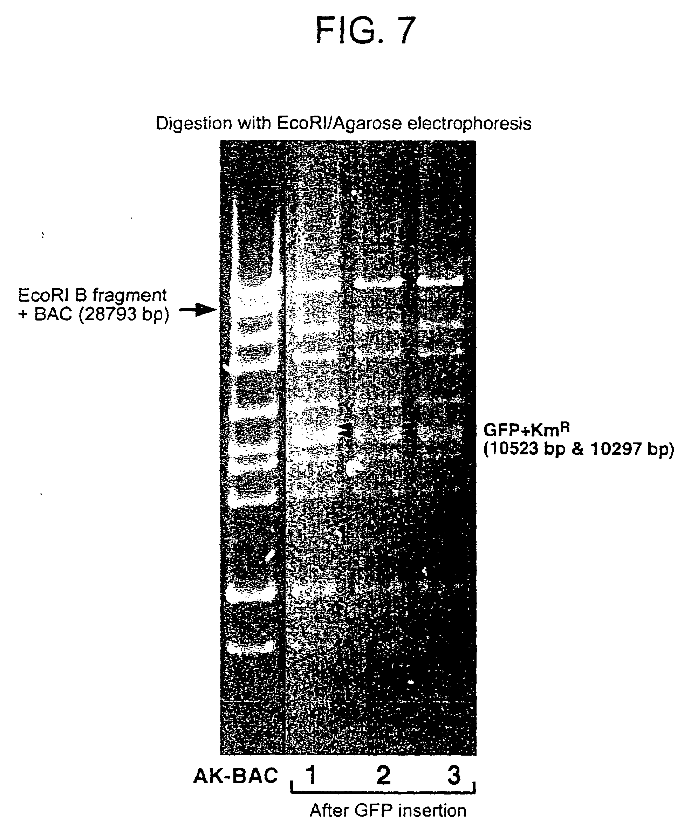

FIG. 7 illustrates the result of agarose gel electrophoresis of the EcoRI-digested DNAs obtained from the colonies. For comparison, EcoRI-digested DNA of AK-BAC prior to homologous recombination (before modification) is shown in the first lane of FIG. 7. A band of 28.8 kb (including the region deleted in B95-8 strain) observed in the lane of AK-BAC before modification disappeared, and instead a new band appeared around 10.5 kb. Southern blotting revealed that this new band was composed of two bands close to each other. Their band sizes (10523 bp and 10297 bp) coincide with those of the bands which are expected to appear by the insertion of a GFP gene and a kanamycin resistant gene (Km^{R}).

These results revealed that the part of the region deleted in B95-8 strain of AK-BAC (FIG. 2) was successfully replaced with a GFP gene by the RecE/RecT-mediated homologous recombination in *Escherichia coli.* All the three clones turned out to be precisely modified by the homologous recombination (FIG. 7).

The obtained BAC clone was designated "AK-BAC-GFP". Forty micrograms of circular DNA of AK-BAC-GFP was prepared from 1 liter of bacterial culture.

### Example 3

In this Example, Akata cells which have lost EB virus (EB virus-negative Akata cells) after long-term passage and subcultivation in culture medium containing 10% bovine fetal serum were used.

The DNA of AK-BAC-GFP obtained in Example 2 was introduced into the EB virus-negative Akata cells by electroporation. Two days later, the transfected cells were suspended in medium containing 700 µg/ml of neomycin. The suspension was seeded in 96-well plates. Half of the culture medium was replaced with fresh neomycin-containing culture medium every 5 days.

Numbers of neomycin-resistant cells obtained after three weeks of selection were analyzed by Southern blotting method, and cell clones harboring AK-BAC-GFP as circular DNAs (episomes) were selected.

The obtained cell clones were subjected to anti-immunoglobulin antibody treatment to induce virus production. Two days later, the culture supernatant was collected, and the amount of the obtained EB virus DNA and the viral titer of transforming cord blood lymphocytes were determined.

The amount of the obtained EB virus DNA was 5 µg per 100 ml of the culture supernatant, and its 50% transformation dose was 10⁷ /ml or higher.

This example has revealed that an infectious recombinant EB virus expressing a GFP protein can be produced by introducing the DNA of AK-BAC-GFP (obtained in Example 2) into EB virus-negative Akata cells by transfection, subjecting the transfected cells to neomycin selection to establish virus-producing cells, and treating the virus-producing cells with an anti-immunoglobulin antibody.

### Example 4

In this Example, Akata cells having circular EB virus genomes (EB virus-positive Akata cells) were employed.

The DNA of AK-BAC-GFP obtained in Example 2 was introduced into EB virus-positive Akata cells by electroporation. Fluorescence microscopic observation revealed that, at 2 days post-transfection, the EB virus-positive Akata cells contained GFP-positive cells at about 10 times more than the EB virus-negative Akata cells of Example 3.

After neomycin selection of the transfected EB virus-positive Akata cells, the obtained cell clones were proliferated and analyzed for the presence of AK-BAC-GFP as circular DNAs (episomes). The result revealed that many of neomycin-resistant cell clones derived from EB virus-positive cells have maintained AK-BAC-GFP as intact episomes. The frequency of the neomycin-resistant cell clones maintaining AK-BAC-GFP as episomes was about 20 times higher than that obtained with EB virus-negative Akata cells of Example 3.

EB virus-positive Akata cell clones having the introduced AK-BAC-GFP as intact episomes were selected, and they were subjected to anti-immunoglobulin antibody treatment to induce virus production. Akata cells having only AK-BAC-GFP were obtained by infecting EB virus negative-Akata cells with the obtained virus solution, and by carrying out neomycin selection. Virus production was induced from the resultant cell clones by anti-immunoglobulin antibody treatment. Two days later, the supernatant was collected from the culture medium, and the amount of the obtained EB virus DNA and the viral titer of transforming cord blood lymphocytes were determined.

The amount of the obtained EB virus DNA was 5 µg per 100 ml of the culture supernatant, and its 50% transformation dose was 10⁷ /ml or higher.

This Example has revealed that cell clones maintaining AK-BAC-GFP as episomes can be established efficiently by introducing AK-BAC-GFP obtained in Example 2 into EB virus-positive Akata cells by transfection, and by carrying out neomycin selection. This example has also revealed that by using viruses produced from the established cell clones for infecting EB virus-negative Akata cells and subsequent neomycin selection result in establishing cell clones which produce infectious recombinant EB viruses expressing GFP protein, without the contamination of wild-type EB virus.

For producing recombinant viruses free of wild-type EB virus, instead of using the infection into EB virus-negative Akata cells, it is also possible to adopt a method of eliminating wild-type EB virus from EB virus-positive Akata cells. Specifically, cell clones which have lost a wild-type EB virus can be isolated by selecting EB virus-positive Akata cell clones having AK-BAC-GFP introduced therein as intact episomes, treating them with hydroxyurea, and then isolating cell clones again. Alternatively, cell clones which have lost a wild-type EB virus can be isolated by selecting EB virus-positive Akata cell clones having AK-BAC-GFP introduced therein as intact episomes, followed by subcultivation and then cloning by the limiting dilution method.

### Example 5

EBNA1 protein is a viral protein essential for the replication and segregation of EB virus genomes as episomes in cell nuclei. The expression of EBNA1 protein is most likely to contribute to the more efficient episome formation in the EB virus-positive Akata cells than in the EB virus-negative Akata cells. Akata cells which constitutively express EBNA1 (EBNA1-expressing Akata cells) were prepared. They were obtained by tranfecting a linear DNA containing an EBNA1 gene and a hygromycin resistant gene into EB virus-negative Akata cells, and then by carrying out hygromycin selection.

Neomycin selection was performed after electroporation of AK-BAC-GFP into the EBNA1-expressing Akata cells. The obtained cell clones were proliferated, and they were analyzed for the presence of AK-BAC-GFP as circular DNAs (episomes). As a result, cell clones harboring AK-BAC-GFP as episomes were obtained at the frequency similar to that obtained with EB virus-positive Akata cells. Virus production was induced from these cell clones. Two days later, the culture supernatant was collected and the amount of the obtained EB virus DNA and the viral titer of transforming cord blood lymphocytes were determined.

The amount of the obtained EB virus DNA was 5 µg per 100 ml of the culture supernatant, and its 50% transformation dose was 10⁷ /ml or higher.

This Example has revealed that, upon re-introduction of AK-BAC-GFP into cells, cell lines maintaining AK-BAC-GFP as episomes can be established efficiently by using EBNA1-expressing Akata cells instead of EB virus-positive Akata cells, and that infectious recombinant EB viruses free of wild-type EB virus contamination can be produced by using the established cell lines.

### Industrial Applicability

The present invention enables modification and proliferation of EB virus circular DNAs in *Escherichia coli* as well as large-scale production of recombinant EB virus virions in Akata cells.

## Claims

1. A double-stranded circular DNA obtainable by inserting a bacterial artificial chromosome vector fragment, derived from an Escherichia coli F factor, into circular Epstein-Barr virus genome.

2. A double-stranded circular DNA according to Claim 1, wherein the circular Epstein-Barr virus genome is derived from Akata cells.

3. A double-stranded circular DNA according to Claim 1 or 2, which has at least one inserted marker gene functioning in cells.

4. A double-stranded circular DNA according to any one of Claims 1 to 3, wherein a desired gene is integrated into the Epstein-Barr virus genome.

5. *Escherichia coli* comprising a double-stranded circular DNA as claimed in any one of Claims 1 to 4 as a bacterial artificial chromosome.

6. A method of using a double-stranded circular DNA as claimed in any one of Claims 1 to 3 as a vector.

7. EBNA1 positive-Akata cells constitutively expressing a viral protein EBNA1, which have been obtainable by introducing a viral gene EBNA1 into Epstein-Barr virus-negative Akata cells.

8. Akata cells obtainable by introducing a double-stranded circular DNA as claimed in Claim 4 into Epstein-Barr virus-positive or -negative Akata cells via transfection.

9. Akata cells obtainable by introducing a double-stranded circular DNA as claimed in Claim 4 into EBNA1-positive Akata cells as claimed in Claim 7 by transfection.

10. A method of producing Epstein-Barr virus, which comprises subjecting Akata cells as claimed in Claim 8 or 9 to anti-immunoglobulin treatment.

11. An Epstein-Barr virus produced from Akata cells as claimed in Claim 8 or 9.

12. A method of producing an Epstein-Barr virus, which comprises:
(a) a step of introducing a double-stranded circular DNA as claimed in Claim 4 into Epstein-Barr virus-positive Akata cells by transfection;
(b) a step of producing an Epstein-Barr virus by anti-immunoglobulin treatment of the Akata cells of (a);
(c) a step of introducing the Epstein-Barr virus produced in the step (b) into Epstein-Barr virus-negative Akata cells;
(d) a step of selecting the Akata cells having the double-stranded circular DNA as claimed in Claim 4 from the infected Akata cells after the step (c); and
(e) a step of subjecting the Akata cells selected in the step (d) to anti-immunoglobulin treatment.

13. A method of producing an Epstein-Barr virus, which comprises:
(a) a step of introducing a double-stranded circular DNA as claimed in Claim 4 into Epstein-Barr virus-positive Akata cells by transfection;
(b) a step of isolating cell clones which have lost a wild-type Epstein-Barr virus from the Akata cells of (a); and
(c) a step of subjecting the cell clones isolated in the step (b) to anti-immunoglobulin treatment.
